# EUROPEAN PATENT APPLICATION

(11) **EP 3 942 927 A1**
(43) Date of publication of application: **26.01.2022**
(21) Application number: 20186949.2
(22) Date of filing: 21.07.2020
(51) Int. Cl.: A01K 29/00, A01K 15/02, A61B 5/024, B68B 1/02, B68B 5/06

(54) **SENSOR ASSEMBLY FOR HORSE MONITORING AND A METHOD OF ATTACHING A SENSOR ASSEMBLY TO A HORSE**

(71) Applicant: Ruxbury ApS, 2970 Hørsholm (DK)
(72) Inventor: GOLDMAN, Tomasz, 2970 Hørsholm (DK); CHRISTENSEN, Leif, 2970 Hørsholm (DK)
(74) Representative: Aera A/S

(57) **Abstract**

A sensor assembly (300) for horse monitoring is disclosed. The sensor assembly comprises a sensor housing (300A). The sensor housing comprises a plurality of surfaces (301-306) including a first surface (301), a second surface (302), a third surface (303), and a fourth surface (304), and one or more protrusions (300C1, 300C2, 300B1, 300B2, 300B3, 300D1, 300D2) including a first primary protrusion (300C1) extending from a surface of the plurality of surfaces. The sensor assembly comprises a first optical sensor (350) positioned at a first window (300B) of the first surface (301) and configured to be positioned below an ear of a horse. The sensor assembly comprises an attachment element (310) comprising a sensor opening (314) configured for alignment with the first window (350). The attachment element comprises one or more openings including a first primary opening (312B) configured to receive the first primary protrusion (300C1). The attachment element is configured to removably attach the sensor assembly (300) to a horse head equipment (12).

## Description

The present disclosure relates to sensor assembly for horse monitoring and a method of attaching a sensor assembly to a horse.

### BACKGROUND

Horses may be subject to several distress situations, where the presence of a caregiver may be vital. For example, colic is one of the most dangerous and costly equine medical problems. Colic is a symptom of a disease and may result in abdominal pain for the horse. The disease, if left untreated, may be fatal for the horse. Further, another distress situation for horses may be if a horse is cast in a stall, which may be a cause of serious injury. Furthermore, foaling when a mare gives birth (to a foal) is another event where the presence of a caregiver may reduce risks to the foal and the mare and prevent complications.

Those events and distress situations may occur at remote locations (for example when the horse is the stall or in a field, far from the owner or caretakers), and often at night.

It is therefore a challenge for horse owners and caretakers to avoid and prevent these events.

Sensor assemblies and monitoring systems for horses exist which allow horse owners or horse personnel to monitor their horses. However, sensor assemblies and monitoring systems for horses may be cumbersome for the horse and the horse equipment and also complicated to install (e.g. mount) on the horse. Often, the horse monitoring systems comprise horse equipment that may be uncomfortable for the horse (such as causing injuries to the horse), which even may have to be removed from the horse (e.g. before going into the stall), and can therefore not be used for monitoring the horse at all time (such as in the stall).

Solutions for monitoring horses exists, where for example fitness products which have been adapted for horses, which use electrocardiogram, ECG, measurement methods to monitor a heart rate of the horse. However, it may not be advisable to use an abdominal belt with sensors, for long time unattended monitoring.

### SUMMARY

There is a need for sensor assemblies for horse monitoring and methods of attaching sensor assemblies to a horse which mitigate, alleviate or address the shortcomings existing and provide an improved monitoring of horses including improved continuous monitoring and improved positioning of the sensor assembly.

A sensor assembly for horse monitoring is provided. The sensor assembly comprises a sensor housing. The sensor housing optionally comprises a plurality of surfaces, e.g. including a first surface, a second surface, a third surface, and a fourth surface. The sensor housing comprises one or more protrusions including a first primary protrusion extending from a surface of the plurality of surfaces. The sensor assembly comprises a first optical sensor positioned at a first window of the first surface and optionally configured to be positioned below an ear of a horse. The sensor assembly comprises an attachment element optionally comprising a sensor opening configured for alignment with the first window. The attachment element comprises one or more openings including a first primary opening configured to receive the first primary protrusion. The attachment element is configured to removably attach the sensor assembly to a horse head equipment.

Further, a method for attaching a sensor assembly to a horse is provided. The method comprises removably attaching the sensor assembly to a horse head equipment. The method comprises positioning the horse head equipment on a head of a horse. The method comprises positioning a first window of the sensor assembly below an ear of the horse.

An advantage of the present disclosure is that an improved attachment of sensor assemblies is provided.

It is an advantage of the present disclosure that an improved monitoring of horses (such as improved continuous monitoring) is provided.

It may be appreciated that the present disclosure provides an improved positioning of the sensor assembly, such as improved positioning of the first optical sensor, which in turn provides improved reliability of measurement including improved precision of measurement, thereby improving the detection of distress situations.

The present disclosure allows for improved comfort for the horse by keeping horse personnel informed of the horse's condition and in particular facilitate fast reactions of the horse personnel to potentially dangerous conditions. For example, positioning the sensor assembly below an ear of the horse may be advantageous since the thickness of the skin of the horse is smaller than at other places of the body of the horse. Further, it may be advantageous to position the sensor assembly below the ear of the horse since the amount of fur at that position may be less than at other places of the body of the horse.

Further, it is an advantage of the present disclosure that a simpler and more flexible mounting of the sensor assembly is provided in turn leading to more precise and accurate positioning of the sensor device. The present disclosure provides a sensor assembly that may be mounted on existing horse head equipment.

### BRIEF DESCRIPTION OF THE DRAWINGS

The above and other features and advantages of the present invention will become readily apparent to those skilled in the art by the following detailed description of exemplary communication devices, methods, and/or server devices thereof with reference to the attached drawings, in which:
Figs. 1A-B are perspective views of an example sensor assembly according to the disclosure seen respectively from the distal side and the proximal side,
Figs. 2A-B are perspective views of an example sensor assembly according to the disclosure, shown without the attachment element, and seen respectively from the proximal side (Fig. 2A) and the distal side (Fig. 2B),
Figs. 3A-C are perspective views of an example sensor assembly according to the disclosure respectively before, while, and after attaching the sensor assembly to a horse head equipment, seen respectively from the distal side and the proximal side,
Fig. 4 is a schematic representation illustrating an example sensor assembly according to the disclosure, positioned below the ear of a horse and attached to a horse a head equipment,
Fig. 5 is a schematic representation illustrating an example scenario of a sensor assembly according to the disclosure, positioned below an ear of a horse at a sensor area,
Fig. 6 is a flow diagram of an exemplary method of attaching a sensor assembly to a horse according to the disclosure, and
Fig. 7 is an exploded view of an exemplary sensor assembly, shown without the attachment element.

### DETAILED DESCRIPTION

Various exemplary methods and/or sensor assemblies and details are described hereinafter, with reference to the figures when relevant. It should be noted that the figures may or may not be drawn to scale and that elements of similar structures or functions are represented by like reference numerals throughout the figures. It should also be noted that the figures are only intended to facilitate the description of the methods and/or sensor assemblies. They are not intended as an exhaustive description of the invention or as a limitation on the scope of the invention. In addition, an illustrated embodiment needs not have all the aspects or advantages shown. An aspect or an advantage described in conjunction with a particular embodiment is not necessarily limited to that embodiment and can be practiced in any other methods and/or sensor assemblies even if not so illustrated, or if not so explicitly described.

In the following, whenever referring to proximal side or surface of a layer, an element, a device or part of a device, the referral is to the fur or skin-facing side or surface of the horse, when a horse wears the sensor assembly. Likewise, whenever referring to the distal side or surface of a layer, an element, a device or part of a device, the referral is to the side or surface facing away from the horse fur, when a horse wears the sensor assembly. In other words, the proximal side or surface is the side or surface closest to the horse, when the sensor assembly is fitted (such as mounted) on a horse head equipment and the distal side is the opposite side or surface - the side or surface furthest away from the horse in use.

The axial direction is defined as the direction of the sensor assembly, when a horse wears the sensor assembly. Thus, the axial direction is generally perpendicular to fur or skin-facing side or surface of the horse.

A radial direction is defined as perpendicular to the axial direction. In some sentences, the words "inner" and "outer" may be used. These qualifiers should generally be perceived with respect to the radial direction, such that a reference to an "outer" element means that the element is farther away from a centre portion of the sensor assembly than an element referenced as "inner". In addition, "innermost" should be interpreted as the portion of a component forming a centre of the component and/or being adjacent to the centre of the component. In analogy, "outermost" should be interpreted as a portion of a component forming an outer edge or outer contour of a component and/or being adjacent to that outer edge or outer contour.

A sensor assembly for horse monitoring is provided.

The first optical sensor may be integrated in a sensor device. The sensor device may have a thickness in the range from 3 mm to 20 mm. The sensor device may have a width in the range from 5 mm to 50 mm and/or a height in the range from 5 mm to 50 mm sensing. The first optical sensor may have a thickness in the range of 2 mm to 10 mm. The first optical sensor may have a width in the range of 5 mm to 30 mm. The first optical sensor may have a height in the range of 5 mm to 30 mm. The sensor assembly comprises a sensor housing. The sensor housing comprises a plurality of surfaces, e.g. including a first surface, a second surface, a third surface, and a fourth surface. The sensor housing comprises one or more protrusions including a first primary protrusion extending from a surface of the plurality of surfaces. In one or more embodiments, the sensor housing comprises a fifth surface and a sixth surface. The term surface may refer to one or more portions of one or more elements that the surface comprises. For example, the first surface may comprise a portion of a first window, a portion of a first optical sensor, and a portion of sensor housing. The one or more protrusions including the first primary protrusion may comprise one or more of a hook, a spike, a dowel, a pin, a nail, a tooth, a peg, and/or a plug.

The sensor assembly comprises a first optical sensor positioned at a first window or first opening of the first surface and configured to be positioned below an ear of a horse. The first window may have an area from 20 mm² to 400 mm².

In one or more embodiments, the sensor housing comprises the first window, such as an opening in the sensor housing (e.g. at the first surface) where the first optical sensor may be positioned. The first window may comprise a hole in the sensor housing, such as a section in the sensor housing, where the first optical sensor may be fitted. The first window may comprise a portion which may not be covered, e.g. by the attachment element, in an attached state of the sensor assembly, such that the first optical sensor may be substantially in contact with the proximal surface. The first window may provide an opening for the first optical sensor, such that the first optical sensor may be able to measure e.g. a heart-rate, a cardiac cycle, a pulse, and/or a blood pressure of the horse, at the proximal surface. The first window may be smaller than the first optical sensor, such that only part of the first optical sensor is covered. The first window may be bigger than the first optical sensor, such that all of the first optical sensor is uncovered. The first window may correspond substantially to the outer dimensions of the first optical sensor, such as fitted to the first optical sensor.

In one or more methods and/or sensor assemblies, positioning the sensor assembly below an ear of the horse comprises to position the sensor assembly in a sensor area. The sensor area may be representative of an area below the ear of the horse where the sensor assembly may be positioned. The term below the ear of the horse may be understood as between the ear of the horse and a temporal bone of the horse, e.g. substantially at the skin surface where an arteria temporalis superficialis is located under the skin. The area below the ear of the horse may be rich in arterial blood (for example close to the arteria temporalis superficialis). The first optical sensor may be configured to be at the proximal surface, such as in contact with the proximal surface. By positioning the sensor assembly below the ear of the horse, an improved reliability of measurement may be achieved. An advantage of positioning the sensor assembly below the ear of the horse may be that the area below the ear of the horse may be rich in arterial blood (for example close to the arteria temporalis superficialis) and the skin and/or fur of the horse may be thinner. The precision of measurement of the sensor assembly and the detection of distress situations may be improved, and in turn also improving the reliability of measurement of the sensor assembly. Furthermore, by positioning the sensor assembly below the ear of the horse, an improved comfort may be provided for the horse, for example substantially avoiding that the horse notices the sensor assembly and is disturbed. Further, by positioning the sensor assembly below the ear of the horse, the sensor assembly may be mounted on existing horse head equipment. This may allow to use the sensor assembly with each horse's own equipment, instead of having to provide special equipment that may be difficult to fit on all horses. This may allow an easier mounting of the sensor assembly with less fitting to each horse for the users.

Horses may have a natural recess below the ear at the temporal (above the temporal bone), where it may be advantageous to position the sensor assembly (for example such that the sensor assembly may be stabilized, e.g. improving the measurement of the sensor assembly).

The first optical sensor may be configured to generate sensor data, such as first sensor data, based on one or more measurements performed on a horse. The sensor data, such as the first sensor data, may comprise and/or be based on one or more first optical signals (such as one or more optical counts measured by the first optical sensor over time). The first optical sensor may comprise one or more optical sources (such as one or more transmitters or emitters of optical signals, for example photo emitters, e.g. LEDs photodiodes), and/or one or more optical receivers (such as photo detectors). The first optical sensor may be configured to measure a heart-rate or other cardiac parameters of the horse.

The first optical sensor may comprise a photoplethysmogram, PPG, sensor. The first optical sensor may be configured to obtain first sensor data, such as obtaining first heart rate data from the first optical sensor (such as from the PPG sensor). The first sensor data may comprise first heart rate data from the first optical sensor. The first heart rate data may comprise one or more electrical signals indicative of a heartbeat of the horse (such as a heart-rate determined for each second, or determined as a median for each minute, measured in Beats per Minute (BPM)), e.g. measured by the first optical sensor for a first time period. The first heart rate data may comprise a plurality of optical counts (such as measured by the first optical sensor, for example by the PPG sensor) over time which may be used to determine horse data, such as used to determine a photoplethysmogram.

In one or more exemplary methods, the sensor assembly comprises a second sensor, and the sensor data comprises second sensor data from the second sensor. In one or more methods and/or sensor assemblies, the sensor assembly may comprise a second sensor and the sensor data may comprise second sensor data from the second sensor.

The sensor assembly comprises an attachment element. The attachment element optionally comprises a sensor opening configured for alignment with the first window. The sensor opening may provide an opening for the first optical sensor, such as an opening at the first surface, such as in alignment with the first window where the first optical sensor may be positioned in an attached state. The sensor opening may comprise a hole in the attachment element for allowing the first optical sensor to be substantially in contact with the proximal surface. The sensor opening may comprise a section in the attachment element, where at least part of the first optical sensor may be uncovered by the attachment element, such that the first optical sensor may be able to measure e.g. a heart-rate or other cardiac parameters of a horse, at the proximal surface, while being securely attached to a horse head equipment. The sensor opening may be smaller than the first window and/or the first optical sensor, such that only part of the first optical sensor and/or the first window are covered by the attachment element. The sensor opening may be larger than the first window and/or the first optical sensor, such that all of the first optical sensor is uncovered. The sensor opening may correspond substantially to the outer dimensions of the first optical sensor and/or the first window, such as fitted to the first optical sensor and/or the first window. The sensor opening may have an area from 20 mm² to 400 mm².

The attachment element comprises one or more openings including a first primary opening configured to receive the first primary protrusion. The one or more openings including the first primary opening may comprise one or more of a hole, a socket, and/or a fitting for receiving one or more protrusions including the first primary protrusion. The one or more protrusions may be inserted in the one or more openings of the attachment element for attaching, such as removably attaching, the attachment element to the sensor housing and/or attaching, such as removably attaching, the sensor assembly to the horse head equipment.

The attachment element is configured to removably attach the sensor assembly to a horse head equipment. The protrusion/opening attachment of the sensor assembly may provide a fast and secure attachment and detachment of the sensor assembly to the horse head equipment. In one or more embodiments, one or more openings may receive one or more protrusions more than one time, e.g. twice, three times, four times etc. For example, when both the attachment element is attached to the sensor housing with one or more openings receiving one or more protrusions and the sensor assembly is attached to the horse head equipment with one or more openings receiving one or more protrusions. In other words, the same protrusion may be inserted in several different openings. For example, when the attachment element is winded around (once or more than once, such as twice) the sensor housing and the horse head equipment. The attachment element may thereby provide a firm attachment to the horse head equipment, such as to an existing horse head equipment (e.g. a halter), for example a firm attachment of the sensor assembly to the skin of the horse. The sensor assembly may thereby be attached to the horse head equipment in a flexible manner, e.g. with a flexible adjustment for a firm attachment of the sensor assembly to the skin and/or fur. This may provide improved conditions for long time measurements by the sensor assembly.

The horse head equipment may be part of a horse head gear, e.g. the horse head gear comprising the horse head equipment and the sensor assembly according to this disclosure. The horse head equipment may comprise one or more of a bridle, a harness, a halter, a cheekpiece, a headpiece, a browband, a throatlatch and a head collar.

In one or more exemplary sensor assemblies, the first surface is a proximal surface, e.g. facing in an opposite direction than the second surface being a distal surface. In other words, the second surface may be distal surface.

In one or more exemplary sensor assemblies, the first primary protrusion extends from the third surface. The third surface and/or the fourth surface may be a side surface with respect to the proximal surface, e.g. the first surface. For example, the first surface being a proximal surface, the second surface being a distal surface, the third surface being a side surface, and the fourth surface being a side surface opposite to the third surface.

In one or more exemplary sensor assemblies, the attachment element may be an elastic strap and optionally comprises a first primary portion configured to extend on a first side (proximal side) of the sensor housing, a second primary portion and/or a second secondary portion configured to extend on a second side (distal side) of the sensor housing, a third primary portion configured to extend on a third side of the sensor housing, and a fourth primary portion configured to extend on a fourth side of the sensor housing. In other words, the attachment element comprises a first primary portion configured to extend on a first side of the sensor housing, a second primary portion and/or a second secondary portion configured to extend on a second side of the sensor housing, a third primary portion configured to extend on a third side of the sensor housing, and a fourth primary portion configured to extend on a fourth side of the sensor housing, when the sensor assembly is in an attached state. In other words, the attachment element may be wound around the sensor housing, e.g. in an attached state. The attachment element may thereby provide support around the sensor housing and in turn provide a secure attachment and positioning of the sensor assembly to the horse head equipment. Furthermore, the attachment element may provide that the first optical sensor is stabilized in its intended measurement position and in turn provides improved reliability of measurement.

In one or more exemplary sensor assemblies, the attachment element comprises a third secondary portion configured to extend on the third side of the sensor housing, the third secondary portion comprising the first primary opening. The third secondary portion may be attached to the sensor housing, e.g. to the first primary protrusion, with the first primary opening. For example, when the attachment element is wound around the sensor housing, the third secondary portion is configured to extend on the third side of the sensor housing. The third secondary portion comprising the first primary opening may thereby secure the sensor assembly to the horse head equipment, when the first primary opening receives the first primary protrusion.

In one or more exemplary sensor assemblies, the one or more protrusions comprise a first secondary protrusion optionally extending from the third surface. The attachment element may comprise a first secondary opening configured to receive the first secondary protrusion. The first secondary protrusion may be a similar protrusion as the first primary protrusion, such as symmetrical to each other, or may be a different protrusion than the first primary protrusion, e.g. different size, different position, and/or different shape. The first secondary protrusion and/or the first secondary opening may provide an improved stability, such as improved stability of the sensor assembly when being attached to the horse head equipment. Furthermore, the first secondary protrusion and/or the first secondary opening may facilitate that movement of the attachment element with regard to the sensor housing is avoided, e.g. by providing a substantially even pull of the attachment element.

In one or more exemplary sensor assemblies, the one or more protrusions comprise a second primary protrusion extending from the second surface. The second primary protrusion may be arranged on one side of the second side, such as on a side furthest away from the first primary protrusion and/or the first secondary protrusion. The second primary protrusion may be a longitudinal protrusion, e.g. extending substantially along a width of the sensor housing, such as having a length substantially equivalent to the width of the sensor housing. The second primary protrusion may for example be arranged such that the horse head equipment e.g. a strap of the horse head equipment may abut the second primary protrusion when the sensor assembly is mounted on the horse head equipment. The second primary protrusion may improve the stability of the attachment of the sensor assembly to the horse head equipment, e.g. avoiding that the horse head equipment moves in relation to the sensor assembly. The second primary protrusion may improve that the sensor assembly remains in the same position, e.g. the same position below the ear of the horse. The second primary protrusion may provide In one or more exemplary sensor assemblies, the sensor assembly comprises a first interface. The first interface may comprise one or more of an antenna, a transceiver, a wired interface, and/or a wireless interface.

The first interface may comprise one or more first primary interfaces (such as one or more wireless interfaces, for example one or more antennas, such as an antenna for using short range wireless communication Bluetooth low energy transmission to the monitor device) and/or one or more first secondary interfaces (such as one or more wired interfaces, for example a USB interface, e.g. for charging the sensor assembly). The first interface may comprise a first tertiary interface and/or a first quaternary interface. In one or more embodiments, the first interface comprises a wired interface comprising one or more connectors (such as terminals) and comprising an interface cover (such as a closing element for the interface connector). The interface cover may for example comprise a rubber plug, e.g. for providing a tight seal (such as sealing the first interface when the interface cover is in the mounted state). In one or more embodiments, the first interface comprises a USB connector (such as a USB terminal), for example comprising a USB cover as the interface cover. The attachment element may for example in an attached state of the sensor assembly, provide a pressure on the interface cover, whereby the interface cover may be pressed towards the sensor housing. This may provide that the interface cover may provide a tight seal against the sensor housing.

The sensor assembly may be configured to transmit, via the first interface, the first sensor data to a monitor device. The monitor device may comprise one or more of: an electronic device (such as a wireless electronic device), a mobile phone, a smartphone, a computer, and/or a gateway. The transmission of the first sensor data may be performed directly to the monitor device or may occur through a relay device forwarding the transmission of the first sensor data to the monitor device. The transmission of the first sensor data to the monitor device may be performed using Bluetooth, for example by communicating with the monitor device directly over Bluetooth or via a Bluetooth gateway (the Bluetooth gateway may then be connected to a global network, such as the internet, for communicating with the monitor device), and/or over mobile network (such as 3G, 4G etc.).

In one or more exemplary sensor assemblies, the second primary protrusion has a hollow portion and wherein the first interface is at least partially arranged in the hollow portion of the second primary protrusion. For example, the first interface such as an antenna may be at least partially arranged in the hollow portion of the second primary protrusion and the remaining of the first interface may be arranged in the sensor housing. By having the first interface arranged at least partially in the hollow portion of the second primary protrusion, the first interface may project outwards from the second surface, e.g. second side, towards the distal side. This may for example allow the first interface to have an improved signal transmission and/or reception, e.g. by being away from the proximal side and away from the remaining elements in the sensor housing. Further, the first interface may also be arranged away from the remaining elements of the sensor assembly in the sensor housing (e.g. as a processor, a memory and/or a battery) to avoid noise and/or interference disturbance between the first interface and the remaining elements.

In one or more exemplary sensor assemblies, the one or more protrusions comprise a third primary protrusion extending from the fourth surface, and wherein the attachment element comprises a fourth primary opening configured to receive the third primary protrusion. The one or more protrusions may comprise a third secondary protrusion extending from the fourth surface, and the attachment element may comprise a fourth secondary opening configured to receive the third secondary protrusion.

The third primary protrusion and/or third secondary protrusion may be a similar protrusion as the first primary protrusion, the first secondary protrusion, and/or the second primary protrusion, such as symmetrical to each other, or may be a different protrusion than the first primary protrusion, the first secondary protrusion, and/or the second primary protrusion e.g. different size, different position, and/or different shape. The third primary protrusion and/or third secondary protrusion and/or the fourth primary opening may provide an improved stability, such as improved stability of the sensor assembly when being attached to the horse head equipment. Furthermore, the third primary protrusion and/or third secondary protrusion and/or the fourth primary opening may avoid movement of the attachment element with regard to the sensor housing, e.g. by providing a substantially even pull of the attachment element. Further, the third primary protrusion and/or third secondary protrusion and/or the fourth primary opening may avoid movement of the sensor opening relative to the first window, e.g. the first optical sensor, e.g. by providing a substantially even pull of the attachment element between the third primary protrusion and/or third secondary protrusion and the first primary protrusion and/or the first secondary protrusion.

In one or more exemplary sensor assemblies, the sensor assembly comprises an attachment mechanism arrangement on the second surface of the sensor housing and configured for attaching an end portion of the attachment element to the sensor housing.

In one or more embodiments, the attachment mechanism arrangement comprises one or more protrusions, such as a second secondary protrusion and/or a second tertiary protrusion extending from the second surface, e.g. the second secondary protrusion being symmetrical to the second tertiary protrusion. The second secondary protrusion and/or the second tertiary protrusion may provide one or more attachment points for the attachment element, such as one or more attachment points for a second secondary opening and/or a second tertiary opening of the attachment element. The second secondary opening and/or the second tertiary opening may receive the second secondary protrusion and/or the second tertiary protrusion, whereby the attachment element may be attached to the sensor housing. In other words, attaching the second primary portion of the attachment element to the sensor housing, such as attaching the second primary portion of the attachment element to the second secondary protrusion and/or the second tertiary protrusion.

In one or more exemplary sensor assemblies, the attachment element comprises a strap with a first end portion and a second end portion, wherein the first end portion is attached to the sensor housing. The second primary portion may comprise the first end portion, such as the first end portion may be part of the second primary portion. In one or more embodiments, the first end portion may be the end portion of the attachment element. The first end portion may be attached to the sensor housing with the attachment mechanism arrangement. In one or more embodiments, the second end portion is attached to the sensor housing. The third secondary portion may comprise the second end portion, such as the second end portion may be part of the third secondary portion. In one or more embodiments, the second end portion may be the end portion of the attachment element.

In other words, the attachment element may comprise a strap, such as a wrap-around elastic strap.

In one or more exemplary sensor assemblies, the strap is an elongated strap made of a rubber material. The strap may also be denominated as a band, a strip, a belt, a strop, and/or a harness. The rubber material may comprise one or more of silicone, neoprene, nitrile, natural rubber, ethylene, and/or propylene. By having a rubber material, the sensor assembly may be attached with the attachment element, e.g. the strap, to the horse head equipment. The strap may provide enough friction, such as an enhanced friction, between the strap and the horse head equipment and/or the proximal surface, e.g. the fur and/or skin of the horse, to securely maintain the sensor assembly in place, even when the horse is in movement. Furthermore, the rubber material mat be chosen such that the strap in comfortable for the horse to wear the sensor assembly, e.g. by being soft for the horse.

The second primary portion and the second secondary portion of the attachment element are arranged on and contacts opposite sides of a portion of the horse head equipment, such as a portion of a halter on the horse. In other words, the portion of the horse head equipment that the sensor assembly is attached to may be sandwiched between the second primary portion and the second secondary portion. The attachment element may therefore provide a secure attachment of the sensor assembly to the horse head equipment and to the proximal surface, e.g. the skin surface. For example, the attachment element may avoid the displacement of the sensor assembly with regard to the horse head equipment, the position where the sensor assembly is positioned (e.g. below the ear of the horse) and/or the attachment element. The attachment element may provide a high friction coefficient around the portion of the horse head equipment that the sensor assembly is attached to.

Further a method for attaching a sensor assembly to a horse is disclosed. The method comprises removably attaching the sensor assembly to a horse head equipment. The method comprises positioning the horse head equipment on a head of a horse. The method comprises positioning a first window of the sensor assembly below an ear of the horse.

In one or more example methods, the horse head equipment may be positioned on the horse first. Then, the sensor assembly may be positioned, such as attached to the horse head equipment. Finally, when the sensor assembly is positioned at a suitable position below the ear of the horse, the sensor assembly may be attached securely, such as tightened to the horse head equipment (e.g. with the attachment element). In some embodiments, the sensor assembly may be attached to the horse head equipment before the horse head equipment is positioned on the horse.

In one or more exemplary methods, removably attaching the sensor assembly to a horse head equipment comprises arranging a housing of the sensor assembly on a proximal side of the horse head equipment, and arranging an attachment element of the sensor assembly on a distal side of the horse head equipment such that the horse head equipment is fastened between the housing and the attachment element.

In one or more embodiments, the sensor assembly is embedded in the horse head equipment, such as embedded in one or more of a bridle, a harness, and a head collar of the horse.

It is to be understood that a description of a feature in relation to sensor assembly(s) is also applicable to the corresponding method(s) and vice versa.

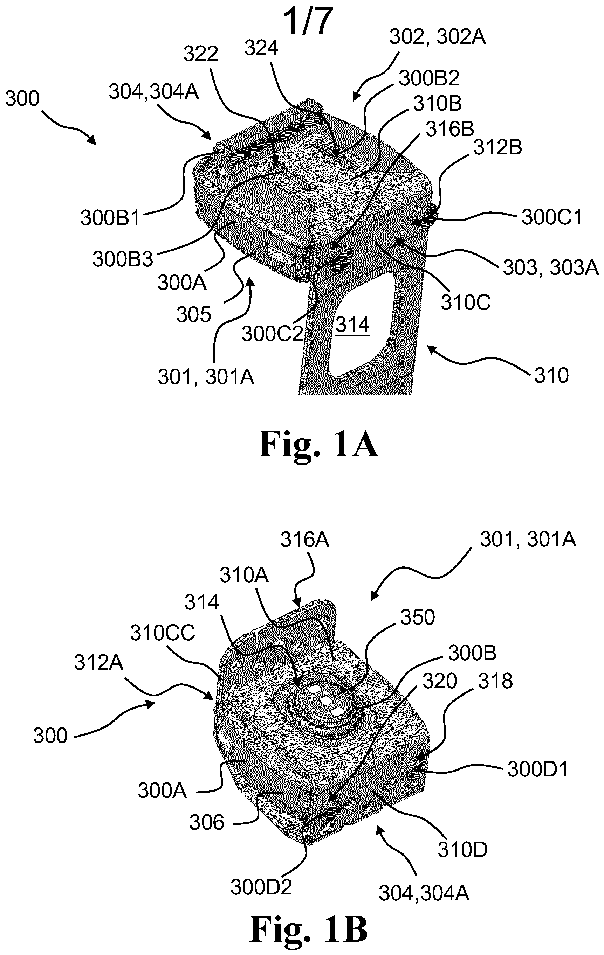

Figs. 1A-B are perspective views of an example sensor assembly according to the disclosure seen respectively from the distal side (Fig. 1A) and the proximal side (Fig. 1B).

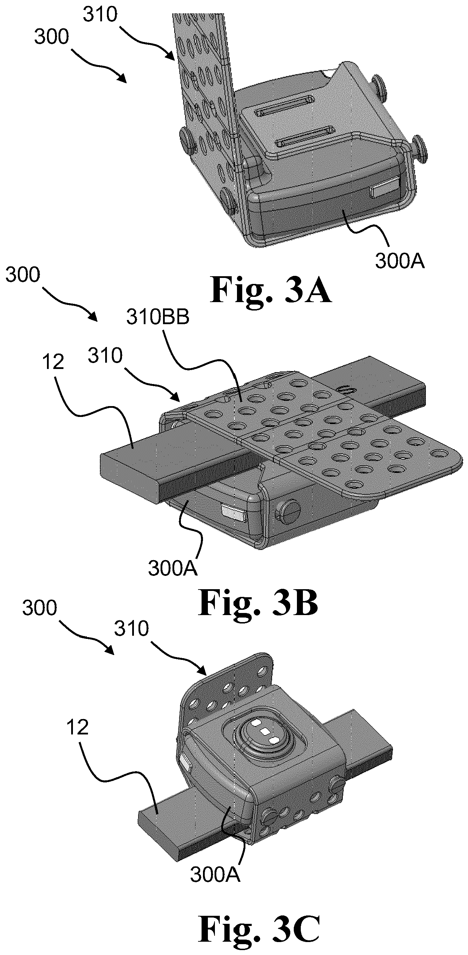

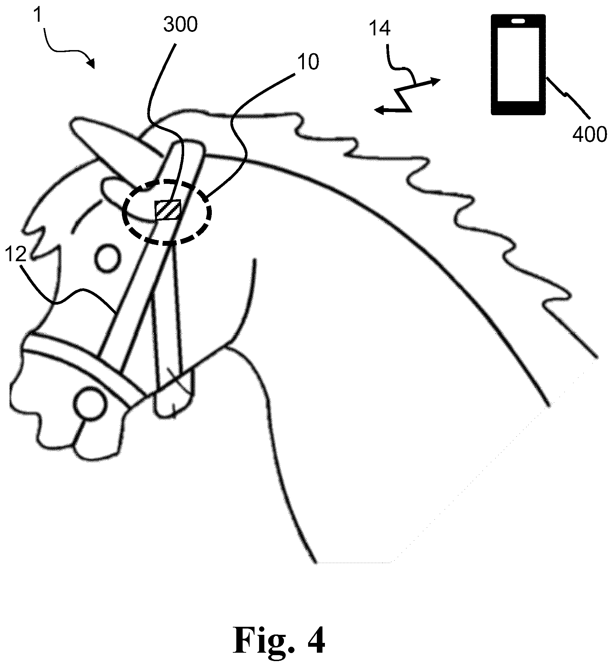

A sensor assembly 300 for horse monitoring is provided. The sensor assembly 300 comprises a sensor housing 300A. The sensor housing 300A comprises a plurality of surfaces including a first surface 301, a second surface 302, a third surface 303, and a fourth surface 304, and one or more protrusions including a first primary protrusion 300C1 extending from a surface of the plurality of surfaces, such as the third surface 303 on Fig. 1A. In one or more embodiments, the sensor housing 300A comprises a fifth surface 305 and a sixth surface 306. The sensor assembly 300 comprises a first optical sensor 350 positioned at a first window 300B of the first surface 301 and configured to be positioned below an ear of a horse. The sensor assembly 300 comprises an attachment element 310 comprising a sensor opening 314 configured for alignment with the first window 300B. The attachment element 310 comprises one or more openings including a first primary opening 312A configured to receive the first primary protrusion 300C1. The attachment element 310 is configured to removably attach the sensor assembly 300 to a horse head equipment 12 (shown in Figs. 3B-C and Fig. 4).

In one or more exemplary sensor assemblies, the attachment element may comprise a plurality of openings. The sensor assembly 300 may thereby be attached to the horse head equipment 12 in a flexible manner, e.g. with a flexible adjustment for a firm attachment of the sensor assembly 300 to the skin and/or fur. The sensor assembly 300 may therefore be adapted to various different horse head equipment of different widths and thicknesses. This may provide improved conditions for long time measurements by the sensor assembly. In one or more exemplary sensor assemblies, the first surface 301 is a proximal surface facing in an opposite direction than the second surface 302 being a distal surface. In one or more exemplary sensor assemblies, the first primary protrusion 300C1 extends from the third surface 303.

In one or more exemplary sensor assemblies, the attachment element 310 comprises a first primary portion 310A configured to extend on a first side 301A of the sensor housing 300A, a second primary portion 310B and/or a second secondary portion 310BB (shown on Fig. 3B) configured to extend on a second side 302A of the sensor housing 300A, a third primary portion 310C configured to extend on a third side 303A of the sensor housing 300A, and a fourth primary portion 310D configured to extend on a fourth side 304A of the sensor housing 300A.

In one or more exemplary sensor assemblies, the attachment element 310 comprises a third secondary portion 310CC configured to extend on the third side 303A of the sensor housing 300A, the third secondary portion 310CC comprising the first primary opening 312A.

In one or more exemplary sensor assemblies, the one or more protrusions comprise a first secondary protrusion 300C2 extending from the third surface 303, and wherein the attachment element 310 comprises a first secondary opening 316A configured to receive the first secondary protrusion 300C2. For example, the third secondary portion 310CC comprises the first secondary opening 316A.

The third primary portion 310C may comprise a third primary opening 312B and/or a third secondary opening 316B. The third primary opening 312B and/or the third secondary opening 316B may be configured to receive the first primary protrusion 300C1 and/or the first secondary protrusion 300C2, such that the third primary portion 310C may be attached to the sensor housing 300A.

In one or more exemplary sensor assemblies, the one or more protrusions comprise a second primary protrusion 300B1 extending from the second surface 302. The second primary protrusion 300B1 is arranged on one side of the second side 302, such as on a side furthest away from the first primary protrusion 300C1 and/or the first secondary protrusion 300C2. The second primary protrusion 300B1 may be a longitudinal protrusion, e.g. extending substantially along a width of the sensor housing 300A, such as having a length substantially equivalent to the width of the sensor housing 300A. The second primary protrusion 300C2 may for example be arranged such that the horse head equipment 12 e.g. a strop of the horse head equipment 12 may abut the second primary protrusion 300C2 (see Fig. 3B). The second primary protrusion 300C2 may improve the stability of the attachment of the sensor assembly 300 to the horse head equipment 12, e.g. avoiding that the horse head equipment 12 moves in relation to the sensor assembly 300. The second primary protrusion 300C2 may improve that the sensor assembly 300 remains in the same position, e.g. the same position below the ear of the horse.

In one or more exemplary sensor assemblies, the sensor assembly 300 comprises a first interface (not shown). The first interface may comprise one or more of an antenna, a transceiver, a wired interface, and/or a wireless interface.

In one or more exemplary sensor assemblies, the second primary protrusion 300B1 has a hollow portion and wherein the first interface is at least partially arranged in the hollow portion of the second primary protrusion 300B1. For example, the first interface such as an antenna may be at least partially arranged in the hollow portion of the second primary protrusion 300B1 and the remaining of the first interface may be arranged in the sensor housing 300A. By having the first interface arranged at least partially in the hollow portion of the second primary protrusion 300B1, the first interface may project outwards from the second surface 302, e.g. second side 302A, towards the distal side. This may for example allow the first interface to have an improved signal transmission and/or reception, e.g. by being away from the proximal side and away from the remaining elements in the sensor housing 300A. Further, the first interface may also be arranged away from the remaining elements of the sensor assembly 300 in the sensor housing 300A (e.g. as a processor, a memory and/or a battery) to avoid noise and/or interference disturbance between the first interface and the remaining elements.

In one or more exemplary sensor assemblies, the one or more protrusions comprise a third primary protrusion 300D1 extending from the fourth surface 304, and wherein the attachment element 310 comprises a fourth primary opening 318 configured to receive the third primary protrusion 300D1. The one or more protrusions may comprise a third secondary protrusion 300D2 extending from the fourth surface 304, and the attachment element 310 may comprise a fourth secondary opening 320 configured to receive the third secondary protrusion 300D2.

In one or more exemplary sensor assemblies, the sensor assembly 300 comprises an attachment mechanism arrangement on the second surface 302 of the sensor housing 300A and configured for attaching an end portion of the attachment element 310 to the sensor housing 300A. In one or more embodiments, the attachment mechanism arrangement comprises one or more protrusions, such as a second secondary protrusion 300B2 and/or a second tertiary protrusion 300B3 extending from the second surface 302, e.g. the second secondary protrusion 300B2 being symmetrical to the second tertiary protrusion 300B3. The second secondary protrusion 300B2 and/or the second tertiary protrusion 300B3 may provide one or more attachment points for the attachment element 310, such as one or more attachment points for a second primary opening 322 and/or a second secondary opening 324 of the attachment element 310. The second primary opening 322 and/or the second secondary opening 324 may receive the second secondary protrusion 300B2 and/or the second tertiary protrusion 300B3, whereby the attachment element 310 may be attached to the sensor housing 300A. In other words, attaching the second primary portion 310B of the attachment element 310 to the sensor housing 300A, such as attaching the second primary portion 310B of the attachment element 310 to the second secondary protrusion 300B2 and/or the second tertiary protrusion 300B3.

In one or more exemplary sensor assemblies, the attachment element 310 comprises a strap with a first end portion and a second end portion, wherein the first end portion is attached to the sensor housing. The second primary portion 310B may comprise the first end portion, such as the first end portion may be part of the second primary portion 310B. In one or more embodiments, the first end portion may be the end portion of the attachment element 310. The first end portion may be attached to the sensor housing 300A with the attachment mechanism arrangement. In one or more embodiments, the second end portion is attached to the sensor housing 300A. The third secondary portion 310CC may comprise the second end portion, such as the second end portion may be part of the third secondary portion 310CC. In one or more embodiments, the second end portion may be the end portion of the attachment element 310.
In one or more exemplary sensor assemblies, the strap is an elongated strap made of a rubber material.

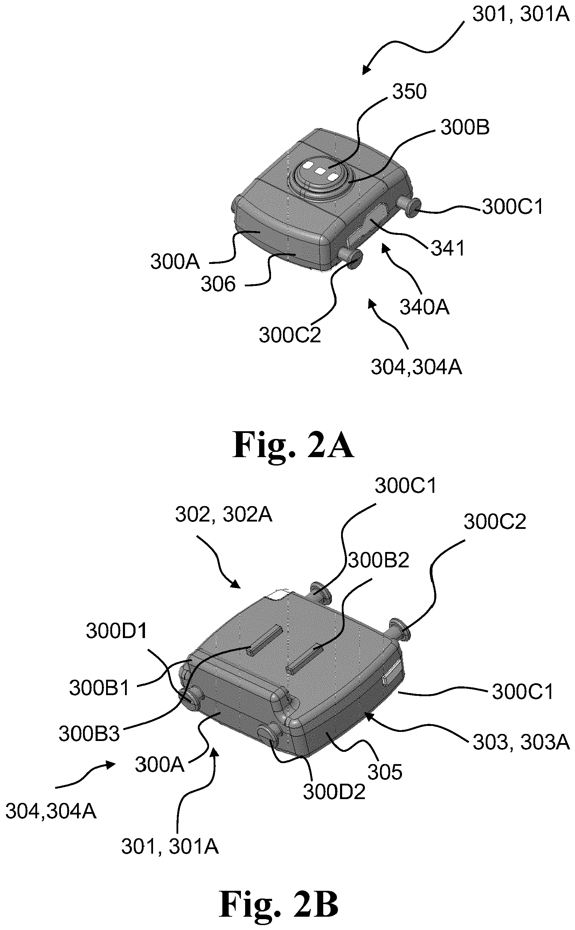

Figs. 2A-B are perspective views of an example sensor assembly 300 according to the disclosure, shown without the attachment element 310, seen respectively from the proximal side (Fig. 2A) and the distal side (Fig. 2B).
The first interface may comprise one or more first primary interfaces (such as one or more wireless interfaces, for example one or more antennas, such as an antenna for using short range wireless communication Bluetooth low energy transmission to the monitor device) and/or one or more first secondary interfaces (such as one or more wired interfaces, for example a USB interface, e.g. for charging the sensor assembly). In one or more embodiments, the first interface comprises a first secondary interface 340A, such as a wired interface comprising one or more connectors (such as terminals) and comprising an interface cover 341 (such as a closing element for the interface connector). The interface cover 341 may for example comprise a rubber plug, e.g. for providing a tight seal (such as sealing the first interface when the interface cover is in the mounted state). In one or more embodiments, the first interface comprises a USB connector (such as a USB terminal), for example comprising a USB cover as the interface cover. The attachment element 310 may for example in an attached state of the sensor assembly 300, provide a pressure on the interface cover 341, whereby the interface cover 341 may be pressed towards the sensor housing 300A. This may provide that the interface cover 341 may provide a tight seal against the sensor housing 300A.

Figs. 3A-C are perspective views of an example sensor assembly 300 according to the disclosure respectively before (Fig. 3A), while (Fig. 3B), and after (Fig. 3C) attaching the sensor assembly 300 to a horse head equipment 12, seen respectively from the distal side (Fig. 3A-B) and the proximal side (Fig. 3C). In Fig. 3A, the attachment element 310 is attached to the sensor housing 300A. In Fig. 3B, the horse head equipment 12 is positioned on the distal side at the distal surface, and the attachment element 310 is positioned on top of a portion of the horse head equipment 12. In Fig. 3C, the sensor assembly 300 is removably attached to the horse head equipment 12 (such as a portion of the horse head equipment 12) with the attachment element 310.

Fig. 4 is a schematic representation illustrating an example sensor assembly 300 according to the disclosure, positioned below the ear of a horse 1 and attached to a horse head equipment 12. The sensor assembly may be located at a sensor area 10.

The sensor assembly 300 may be configured to transmit 14 sensor data to a monitor device 400. For example, a user (such as a horse owner, a horse trainer, a horse rider, or a person taking care of the horse) may monitor the horse 1 on the monitor device 400 by receiving sensor data on the monitor device. The user monitoring the horse 1 may for example be able to receive information about the horse 1 during a training of the horse 1 (such as while riding the horse or on the ground next to the horse) or while the horse is in the stable (such as monitoring a level of activity of the horse during the day or the night, a stress level of the horse, a health condition of the horse, whether the horse is sexually receptive, pregnant, or in gestation). The sensor assembly 300 may transmit (such as report) to the monitor device 400, for notifying the owner or caregiver to attend the distressed horse. For example, the sensor data may comprise a heart rate of the horse, which may be indicative (such as a key indicator) of distress of the horse (such as foaling), e.g. if the horse has a unusually high heart rate (for example an unusually high heart rate when being in the stall, or at an unusual time).

In Fig. 4, the sensor assembly 300 is positioned in a sensor area 10. The sensor area 10 may be representative of an area below the ear of the horse 1 where the sensor assembly 300 may be positioned. In Fig. 4, the sensor assembly 300 is shown for illustrative purposes above (such as on top of) a horse head equipment 12. In practice (such as in use, for example a mounted state of the sensor assembly 300), the sensor assembly 300 may be positioned below the horse head equipment 12 (for example removably attached to the horse head equipment 12, e.g. with the attachment element 310 such as the strap) in contact with a proximal surface, such as a skin surface of the horse.

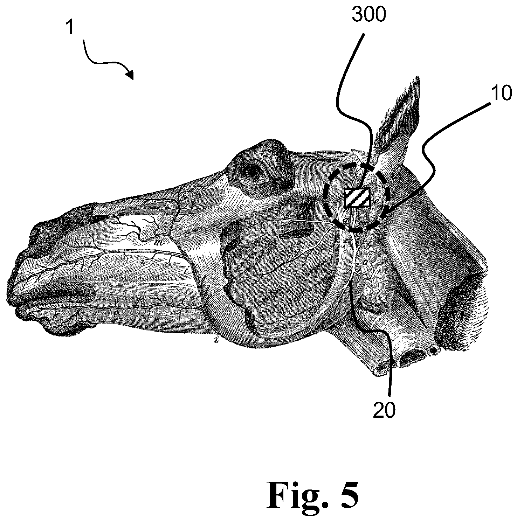

Fig. 5 is a schematic representation illustrating an example scenario of a sensor assembly 300 according to the disclosure, positioned below an ear of a horse 1 at a sensor area 10. The sensor area 10 is located at a first portion of an artery 20 of the horse 1 (such as the sensor area 10 may be representative of a first area located at the first portion of the artery 20). In one or more exemplary methods and/or sensor assemblies, the artery 20 may be an arteria temporalis superficialis of the horse 1. In one or more exemplary methods and/or sensor assemblies, the sensor assembly 300 may be configured to be positioned at a second portion of the artery 20 of the horse 1 (such as the sensor area 10 may be representative of a second area located at the second portion of the artery 20), and/or at a third portion of the artery 20 of the horse 1 (such as the sensor area 10 may be representative of a third area located at the third portion of the artery 20), for example depending on the horse head equipment that the horse is wearing, such as where on the horse head equipment the sensor assembly 300 may be attached and at which portion of the artery 20 the sensor assembly 300 is positioned (for example at which area the sensor assembly 300 is positioned). In one or more methods and/or sensor assemblies, positioning the sensor assembly 300 below an ear of the horse 1 comprises to position the sensor assembly 300 in a sensor area 10. The sensor area 10 may be representative of an area below the ear of the horse 1 where the sensor assembly 300 may be positioned. The term below the ear of the horse 1 may be understood as between the ear of the horse and a temporal bone of the horse, e.g. substantially at the skin surface where an arteria temporalis superficialis 20 is located under the skin. The area below the ear of the horse 1 may be rich in arterial blood (for example close to the arteria temporalis superficialis 20). The first optical sensor 350 may be configured to be at the proximal surface, such as in contact with the proximal surface. By positioning the sensor assembly 300 below the ear of the horse 1, an improved reliability of measurement may be achieved. An advantage of positioning the sensor assembly 300 below the ear of the horse 1 may be that the area below the ear of the horse may be rich in arterial blood (for example close to the arteria temporalis superficialis 20) and the skin and/or fur of the horse may be thinner. The precision of measurement of the sensor assembly and the detection of distress situations may be improved, and in turn also improving the reliability of measurement of the sensor assembly 300. Furthermore, by positioning the sensor assembly 300 below the ear of the horse 1, an improved comfort may be provided for the horse 1, for example substantially avoiding that the horse notices the sensor assembly 300 and is disturbed. Further, by positioning the sensor assembly 300 below the ear of the horse, the sensor assembly 300 may be mounted on existing horse head equipment with the attachment element. This may allow to use the sensor assembly 300 with each horse's own equipment, instead of having to provide special equipment that may be difficult to fit on all horses. This may allow an easier mounting of the sensor assembly 300 with less fitting to each horse for the users.

Horses may have a natural recess below the ear at the temporal (above the temporal bone), where it may be advantageous to position the sensor assembly (for example such that the sensor assembly may be stabilized, e.g. improving the measurement of the sensor assembly).

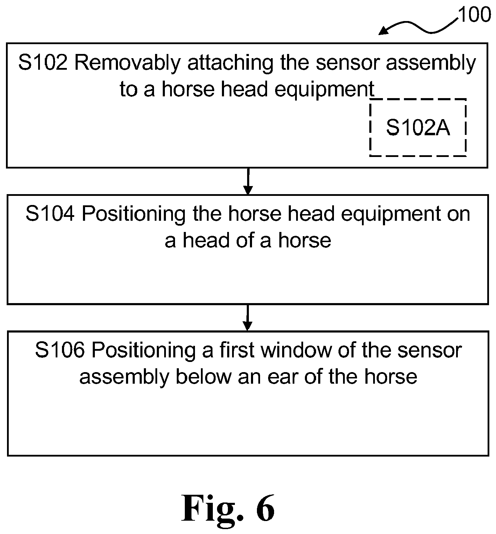

Fig. 6 is a flow diagram of an exemplary method 100 of attaching a sensor assembly (such as sensor assembly 300 of Figs. 1A-B, 2A-B, 3A-C, 4, and 5) to a horse according to the disclosure. The method 100 comprises removably attaching S102 the sensor assembly to a horse head equipment and positioning S104 the horse head equipment on a head of a horse. The horse head equipment may be positioned on a head of a horse prior to or after removably attaching S102 the sensor assembly to the horse head equipment. The method 100 comprises positioning S106 a first window of the sensor assembly below an ear of the horse.

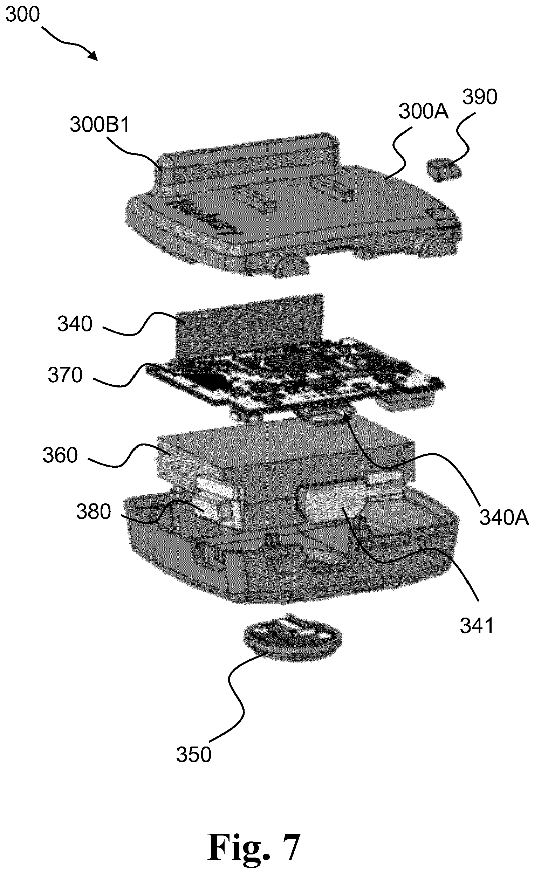

In one or more exemplary methods, removably attaching S102 the sensor assembly to a horse head equipment comprises arranging S102A a sensor housing of the sensor assembly on a proximal side of the horse head equipment, and arranging an attachment element of the sensor assembly on a distal side of the horse head equipment such that the horse head equipment is fastened between the sensor housing and the attachment element.Fig. 7 is an exploded view of an exemplary sensor assembly 300, shown without the attachment element.

The sensor assembly 300 comprises a first primary interface 340. The first primary interface 340 comprises an antenna 340.

In one or more exemplary sensor assemblies, the second primary protrusion 300B1 has a hollow portion and wherein the first primary interface 340 is at least partially arranged in the hollow portion of the second primary protrusion 300B1. For example, the first interface such as the antenna may be at least partially arranged in the hollow portion of the second primary protrusion 300B1 and the remaining of the first interface may be arranged in the sensor housing 300A. By having the first primary interface 340 arranged at least partially in the hollow portion of the second primary protrusion 300B1, the first interface 340 may project outwards from the second surface 302, e.g. second side 302A, towards the distal side. This may for example allow the first primary interface 340 to have an improved signal transmission and/or reception, e.g. by being away from the proximal side and away from the remaining elements in the sensor housing 300A. Further, the first primary interface 340 may also be arranged away from the remaining elements of the sensor assembly 300 in the sensor housing 300A (e.g. as a circuit board 370, a memory, a first secondary interface 340A, the first optical sensor 350, and/or a battery 360) to avoid noise and/or interference disturbance between the first interface and the remaining elements. The sensor assembly may comprise a first tertiary interface 380, such as an on/off button. The sensor assembly may comprise a first quaternary interface 390, such as a light indicator, for example indicating the state that the sensor assembly is in. For example, the light indicator may indicate one or more of the following statuses: on, off, active, inactive, battery status, transmitting, and/or receiving.

The operations of the sensor assembly 300 may be embodied in the form of executable logic routines (for example, lines of code, software programs, etc.) that are stored on a non-transitory computer readable medium (for example, a memory circuitry of the sensor assembly) and are executed by a processor circuitry of the sensor assembly).

Furthermore, the operations of the sensor assembly 300 may be considered a method that the sensor assembly 300 is configured to carry out. Also, while the described functions and operations may be implemented in software, such functionality may as well be carried out via dedicated hardware or firmware, or some combination of hardware, firmware and/or software.

Memory circuitry may be one or more of a buffer, a flash memory, a hard drive, a removable media, a volatile memory, a non-volatile memory, a random access memory (RAM), or other suitable device. In a typical arrangement, memory circuitry may include a non-volatile memory for long term data storage and a volatile memory that functions as system memory for processor circuitry. Memory circuitry may exchange data with processor circuitry over a data bus. Memory circuitry is considered a non-transitory computer readable medium.

Memory circuitry may be configured to store information (such as information indicative of the sensor data, the horse data, the first sensor data, the second sensor data, the first heart rate data, the PPG) in a part of the memory.

It is to be understood that a description of a feature in relation to sensor assembly(s) is also applicable to the corresponding method(s), and vice versa.

The use of the terms "first", "second", "third" and "fourth", "primary", "secondary", "tertiary" etc. does not imply any particular order, but are included to identify individual elements. Moreover, the use of the terms "first", "second", "third" and "fourth", "primary", "secondary", "tertiary" etc. does not denote any order or importance, but rather the terms "first", "second", "third" and "fourth", "primary", "secondary", "tertiary" etc. are used to distinguish one element from another. Note that the words "first", "second", "third" and "fourth", "primary", "secondary", "tertiary" etc. are used here and elsewhere for labelling purposes only and are not intended to denote any specific spatial or temporal ordering.

Furthermore, the labelling of a first element does not imply the presence of a second element and vice versa. It may be appreciated that Figs. 1A-7 comprise some modules or operations which are illustrated with a solid line and some modules or operations which are illustrated with a dashed line. The modules or operations which are comprised in a solid line are modules or operations which are comprised in the broadest example embodiment. The modules or operations which are comprised in a dashed line are example methods and/or sensor assemblies which may be comprised in, or a part of, or are further modules or operations which may be taken in addition to the modules or operations of the solid line example methods and/or sensor assemblies. It should be appreciated that these operations need not be performed in order presented. Furthermore, it should be appreciated that not all of the operations need to be performed. The exemplary operations may be performed in any order and in any combination.

It is to be noted that the word "comprising" does not necessarily exclude the presence of other elements or steps than those listed.

It is to be noted that the words "a" or "an" preceding an element do not exclude the presence of a plurality of such elements.

It should further be noted that any reference signs do not limit the scope of the claims, that the exemplary methods and/or sensor assemblies may be implemented at least in part by means of both hardware and software, and that several "means", "units" or "devices" may be represented by the same item of hardware.

The various exemplary methods and devices described herein are described in the general context of method steps processes, which may be implemented in one aspect by a computer program product, embodied in a computer-readable medium, including computer-executable instructions, such as program code, executed by computers in networked environments. A computer-readable medium may include removable and non-removable storage devices including, but not limited to, Read Only Memory (ROM), Random Access Memory (RAM), compact discs (CDs), digital versatile discs (DVD), etc. Generally, program modules may include routines, programs, objects, components, data structures, etc. that perform specified tasks or implement specific abstract data types. Computer-executable instructions, associated data structures, and program modules represent examples of program code for executing steps of the methods disclosed herein. The particular sequence of such executable instructions or associated data structures represents examples of corresponding acts for implementing the functions described in such steps or processes.

Although features have been shown and described, it will be understood that they are not intended to limit the claimed invention, and it will be made obvious to those skilled in the art that various changes and modifications may be made without departing from the spirit and scope of the claimed invention. The specification and drawings are, accordingly, to be regarded in an illustrative rather than restrictive sense. The claimed invention is intended to cover all alternatives, modifications, and equivalents.

### LIST OF REFERENCES

1 horse
10 sensor area
12 horse head equipment
14 transmit
20 artery
300 sensor assembly
300A sensor housing
300B first window
300B1 second primary protrusion
300B2 second secondary protrusion
300B3 second tertiary protrusion
300C1 first primary protrusion
300C2 first secondary protrusion
300D1 third primary protrusion
300D2 third secondary protrusion
301 first surface
301A first side
302 second surface
302A second side
303 third surface
303A third side
304 fourth surface
304 fourth side
305 fifth surface
306 sixth surface
310 attachment element
310A first primary portion
310B second primary portion
310BB second secondary portion
310C third primary portion
310CC third secondary portion
310D fourth primary portion
312A first primary opening
312B third primary opening
314 sensor opening
316A first secondary opening
316B third secondary opening
318 fourth primary opening
320 fourth secondary opening
322 second primary opening
324 second secondary opening
340 first primary interface
340A first secondary interface
350 first optical sensor
360 battery
370 circuit board
380 first tertiary interface
390 first quaternary interface
400 monitoring device
100 method of attaching a sensor assembly to a horse
S102 attaching the sensor assembly to a horse head equipment
S102A arranging a sensor housing of the sensor assembly on a proximal side of the horse head equipment, and arranging an attachment element of the sensor assembly on a distal side of the horse head equipment such that the horse head equipment is fastened between the sensor housing and the attachment element
S104 positioning the horse head equipment on a head of a horse
S106 positioning a first window of the sensor assembly below an ear of the horse

## Claims

1. A sensor assembly for horse monitoring, the sensor assembly comprising:
- a sensor housing comprising:
∘ a plurality of surfaces including a first surface, a second surface, a third surface, and a fourth surface, and
∘ one or more protrusions including a first primary protrusion extending from a surface of the plurality of surfaces;
- a first optical sensor positioned at a first window of the first surface and configured to be positioned below an ear of a horse; and
- an attachment element comprising a sensor opening configured for alignment with the first window, the attachment element comprising one or more openings including a first primary opening configured to receive the first primary protrusion, the attachment element being configured to removably attach the sensor assembly to a horse head equipment.

2. Sensor assembly according to claim 1, wherein the first surface is a proximal surface facing in an opposite direction than the second surface being a distal surface.

3. Sensor assembly according to any of the preceding claims, wherein the first primary protrusion extends from the third surface.

4. Sensor assembly according to any of the preceding claims, wherein the attachment element comprises a first primary portion configured to extend on a first side of the sensor housing, a second primary portion and/or a second secondary portion configured to extend on a second side of the sensor housing, a third primary portion configured to extend on a third side of the sensor housing, and a fourth primary portion configured to extend on a fourth side of the sensor housing.

5. Sensor assembly according to any of the preceding claims, wherein the attachment element comprises a third secondary portion configured to extend on the third side of the sensor housing, the third secondary portion comprising the first primary opening.

6. Sensor assembly according to any of the preceding claims, wherein the one or more protrusions comprise a first secondary protrusion extending from the third surface, and wherein the attachment element comprises a first secondary opening configured to receive the first secondary protrusion.

7. Sensor assembly according to any of the preceding claims, wherein the one or more protrusions comprise a second primary protrusion extending from the second surface.

8. Sensor assembly according to any of the preceding claims, wherein the sensor assembly comprises a first interface.

9. Sensor assembly according to claim 8 as dependent on claim 7, wherein the second primary protrusion has a hollow portion and wherein the first interface is at least partially arranged in the hollow portion of the second primary protrusion.

10. Sensor assembly according to any of the preceding claims, wherein the one or more protrusions comprise a third primary protrusion extending from the fourth surface, and wherein the attachment element comprises a fourth primary opening configured to receive the third primary protrusion.

11. Sensor assembly according to any of the preceding claims, wherein the sensor assembly comprises an attachment mechanism arrangement on the second surface of the sensor housing and configured for attaching an end portion of the attachment element to the sensor housing.

12. Sensor assembly according to any of the preceding claims, wherein the attachment element comprises a strap with a first end portion and a second end portion, wherein the first end portion is attached to the sensor housing.

13. Sensor assembly according to claim 12 wherein the strap is an elongated strap made of a rubber material.

14. Method of attaching a sensor assembly to a horse, the method comprising:
- removably attaching the sensor assembly to a horse head equipment;
- positioning the horse head equipment on a head of a horse; and
- positioning a first window of the sensor assembly below an ear of the horse.

15. Method according to claim 14, wherein removably attaching the sensor assembly to a horse head equipment comprises arranging a sensor housing of the sensor assembly on a proximal side of the horse head equipment, and arranging an attachment element of the sensor assembly on a distal side of the horse head equipment such that the horse head equipment is fastened between the sensor housing and the attachment element.
